# EUROPÄISCHE PATENTSCHRIFT

(11) **EP 1 395 295 B1**
(45) Veröffentlichungstag und Bekanntmachung des Hinweises auf die Patenterteilung: **30.03.2005**
(21) Anmeldenummer: 02748595.2
(22) Anmeldetag: 14.06.2002
(51) Int. Cl.: A61L 24/00, A61L 27/46, A61L 27/44, A61K 6/033, A61K 6/083, A61L 24/04

(54) **TEMPORÄRES ADHÄSIV FÜR METALL-METALL- UND METALL-KERAMIK-BINDUNGEN**
TEMPORARY ADHESIVE FOR METAL - METAL AND METAL-CERAMIC BONDS
ADHESIF TEMPORAIRE POUR DES LIAISONS METAL-METAL ET METAL-CERAMIQUE

(30) Priorität: 15.06.2001 DE 10129845
(43) Veröffentlichungstag der Anmeldung: 10.03.2004
(73) Patentinhaber: Humboldt Universität Berlin, 10117 Berlin (DE); BAM Bundesanstalt für Materialforschung und -prüfung, 12205 Berlin (DE); Vita Zahnfabrik H. Rauter GmbH & Co. KG, 79704 Bad Säckingen (DE)
(72) Erfinder: MÜLLER, Wolf-Dieter, 12524 Berlin (DE); BERGER, Georg, 16341 Zepernick (DE); NAGEL, Emil, 79713 Bad Säckingen (DE); LANGE, Klaus-Peter, 13086 Berlin (DE)
(74) Vertreter: Walter, Wolf-Jürgen
(86) Internationale Anmeldenummer: PCT/DE2002/002227
(87) Internationale Veröffentlichungsnummer: WO 2002/102426

(56) Entgegenhaltungen:
- EP-A- 0 383 595
- EP-A- 0 434 010
- WO-A-01/41713
- DE-A- 2 518 153
- DE-A- 2 906 413
- DE-A- 19 641 775
- DE-A- 19 963 251
- DE-C- 19 744 809
- GB-A- 1 091 476
- US-A- 5 476 880
- US-A- 5 968 999

## Beschreibung

Die Erfindung betrifft ein Verfahren zur Herstellung eines Adhäsivs und ein Adhäsiv-Kit zur Verbindung gleicher oder verschiedener Metallflächen oder Keramiken, insbesondere im biomedizinischen Bereich.

Bekannt sind Knochenzemente für die Gelenkverankerung und für sonstige Knochendefekte, aus einem Kunststoff bestehen, in der Regel basierend auf Methylmethacrylat bzw. verwandter Substanzen, teilweise unter Zusatz weiterer Ester der Acryl- bzw. Methacrylsäure. Solche Knochenzemente sind z.B. in der DE 196 41 775 A1 beschrieben. Häufig wird dabei die Kombination Benzoylperoxid/Dimethyl-p-Toluidin als Katalysator im flüssigen Monomer verwendet, wie in der DE 196 35 205 nachteilig herausgestellt. üblicherweise werden Knochenzemente aus zwei Komponenten angemischt. Eine Komponente enthält das flüssige Monomer, die andere Komponente ein pulverförmiges Polymerisat, das in Form kugelförmiger Partikel mit einem Durchmesser von ca. 100 µm vorliegt.

Derartige Knochenzemente oder andere beispielsweise in der Stomatologie verwendete Klebemittel sind auf sehr lange Haltbarkeiten eingestellt und lassen eine zwischenzeitliche Lösung der Klebeverbindung z.B. zur Inspektion im Regelfall nicht zu.

Ein weitere grundsätzliches Problem bei zu polymerisierenden Materialien besteht darin, daß während der Polymerisation exotherm Wärme freigesetzt wird. Steigt die dabei auftretende Temperatur über 50°C an, werden die im Kontakt befindlichen Knochenzellen geschädigt. Die tatsächliche thermische Beanspruchung von Körperzellen an der Kontaktzone zum polymerisierenden Knochenzement ist nur mit großer Ungenauigkeit vorherzusagen. Sie hängt von der Dicke der eingebrachten Schicht und der Wärmeleitfähigkeit über die Prothesenkomponenten und vom Knochen ab. Laborversuche zeigten, daß unter bestimmten Bedingungen mit handelsüblichen Zementen während der Polymerisation Maximaltemperaturen von bis zu 110°C auftreten können, so daß Verbrennungen als Folgeerscheinungen zu beobachten sind. Hier erscheinen Verbesserungen notwendig.

Ein weiteres Problem der bisher bekannten Knochenzemente ist damit verbunden, daß sowohl stets vorhandenes restliches Monomer als auch andere Zusätze wie der Stabilisator Hydrochinon (Giftklasse 3) sowie der Akzelerator N,N-Dimethyl-p-toluidin (Giftklasse 2) herausgelöst und damit schädigend wirken können.

Der Erfindung liegt die Aufgabe zugrunde, bisherige polymerisationsbedingte Komponenten oder Wirkungen bei Klebeverbindungen im biomedizinischen Bereich zu vermeiden und zugleich eine temporäre biokompatible Bindung zwischen Metall und Metall oder Metall und Keramik zu ermöglichen.

Das erfindungsgemäße Verfahren zur Herstellung eines temporären Adhäsivs löst die o.g. Probleme, indem erst einmal die Polymerisation als solche bei der Bildung des Adhäsivs vermieden wird und zum zweiten ein spezielles bioaktives Material zugesetzt wird. Erfindungsgemäß besteht das Verfahren darin, daß man 15 bis 50 Gew-% eines monomerfreien Polymethylmethacrylates (PMMA) mit einer mittleren Molmasse von 3000 bis 200000 Dalton und einer Säurezahl von 10 bis 350 mg KOH pro g Polymer mit einem biologisch verträglichen, organischen Lösungsmittel oder Lösungsmittelgemisch für das PMMA vermischt, und der Mischung 0,05 bis 80 Gew-% eines bioaktiven, glasig-kristallinen Materials mit einer Korngröße im Bereich von 0,05 bis 20

Die DE-OS 2518153 beschreibt eine zur Verankerung von Implantaten dienende Mischung, die aus einem Polymeren, einem flüssigen Monomeren, gegebenenfalls weiteren Zusätzen und einem biologisch verträglichen Gel besteht. Die GB 1091476 betrifft eine knetbare Dentalfüllmasse aus Polymethylmetacrylat gelöst in Ethylacetat, Zinkoxid und Gips, bei der eine Aushärtung mit dem Gips erfolgt.

Die DE 19744980 C1 beschreibt einen glasig-kristallinen Formkörper mit schneller Löslichkeit und mit den Hauptkristallphasen Calcium-Natrium-Kalium-Phosphat, wobei dieser Formkörper zusätzlich Bor enthält. µm unter Rühren und bei einer Temperatur von 10 bis 50 °C zusetzt bis zum Erhalt einer fließfähigen Mischung mit einer offenen Verarbeitungszeit im Bereich von 1 bis 20 Minuten, wobei das glasig-kristalline Material aus 15 - 45 Gew-% CaO, 40 - 45 Gew-% P₂O₅, 10 - 40 Gew-% ZrO₂ und 0,7 - 3,5 Gew-% Fluorid besteht und als Hauptkristallphasen Apatit und Calciumzirconiumphosphat und als Nebenbestandteil eine Glasphase enthält, worin die Hauptkristallphasen zusammen wenigstens 35 Gew-% betragen und die Nebenbestandteile 5 bis 15 Gew-% betragen.

Dem Gemisch kann weiterhin 3 bis 30 Gew-% eines biologisch verträglichen zinkhaltigen Pulvers zugesetzt werden und/oder ein vollständig oder teilweise resorbierbares biokeramisches Material.

Durch den Verzicht auf eine innerhalb der Mischung ablaufende Polymerisationsreaktion kann ein Einsetzen und Aushärten bei Körpertemperatur realisiert werden. Zu diesem Zweck wird ein säurezahlmodifiziertes PMMA entsprechender Molmasse in einem dafür geeigneten Lösungsmittel, z.B. Acetylessigsäureethylester oder Mischungen von Acetylessigsäureethylester mit Ethanol, der bis zu 4 Vol-% Wasser enthalten kann. Die sich ergebende klebrige, fließende Komponente wird nun mit einem Pulvergemisch aus dem glasig-kristallinen Material und gegebenenfalls aus z.B. ZnO und vollständig oder teilweise resorbierbarer und/oder langzeitstabiler Biokeramik sowie gegebenenfalls TiO₂ vermengt. Dabei haben alle pulverförmigen Bestandteile Korngrößen im Bereich von 0,005 bis 20 µm. Im Ergebnis dieser Prozedur entsteht ex vivo eine fließfähige, spritzbare und streichbare Masse, die über einen Zeitraum von einigen Minuten, z.B. 1 - 10 min, in Abhängigkeit vom Pulveranteil, verarbeitet werden kann.

Bevorzugt wird ein Polymethylmethacrylat mit einem Anteil von 30 bis 35 Gew-% eingesetzt.

Die mittler Molmasse des PMMA kann vorteilhaft im Bereich von 20000 und 80000 Dalton liegen.

Die Säurezahl kann vorteilhaft im Bereich von 25 bis 65 mg KOH pro g Polymer liegen. Die Säurezahl gibt in diesem Zusammenhang die Anzahl der mg KOH an, die zur Neutralisation von 1 g der Polymerprobe verbraucht wird. Sie ist ein wesentliches Kriterium, da die Anzahl der freien Carboxylgruppen am Polymeren für die Bindung der Metallkomponenten wichtig ist.

Das säurezahlmodifizierte Acrylat kann hergestellt werden aus Methylmethacrylat und Methacrylsäure in einer Suspensionspolymerisation, wobei das Verhältnis der Molmasse so gewählt werden muß, daß die gewünschte Säurezahl erhalten wird. Desweiteren wird das säurezahlmodifizierte Polymere durch alkalische Verseifung eines Polymeren aus Methylmethacrylat und Ethylmethacrylat erhalten. Der Anteil des Ethylmethacrylates liegt zwischen 2 und 10 Mol, vorzugsweise bei 6 Mol.

Ein bevorzugtes glasig-kristallines Material enthält 23 - 39 Gew-% CaO, 40 - 45 Gew-% P₂O₅, 20 - 35 Gew-% ZrO₂ und 1 - 6 Gew-% Fluorid und enthält als Hauptkristallphasen Apatit und Calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen.

Ein ebenfalls bevorzugtes glasig-kristallines Material enthält 23 - 39 Gew-% CaO, 40 - 45 Gew-% P₂O₅, 20 - 35 Gew-% ZrO₂ und 1 - 3 Gew-% Fluorid sowie zusätzlich 0,1 bis 6 Gew-% Na₂O, und es enthält als Hauptkristallphasen Apatit und Calciumzirconiumphosphat und als Nebenbestandteil eine Glasphase und zusätzlich als Nebenbestandteil eine Natriumzirconiumphosphat-Phase. Dabei beträgt der Anteil der Hauptkristallphasen zusammen wenigstens 35 Gew-%, und die Nebenbestandteile können jeweils 5 bis 15 Gew-% betragen.

Weiterhin kann das erfindungsgemäße glasig-kristalline Material zusätzlich 0,1 bis 6 Gew-% Magnesiumoxid und/oder Kaliumoxid enthalten und zusätzlich auch die entsprechenden Phasen.

Der Gehalt an Na₂O, MgO und/oder K₂O liegt vorzugsweise im Bereich von 1 bis 6 Gew-%. Der Anteil der entsprechenden Nebenkristallphase Natriumzirconiumphosphat liegt vorzugsweise im Bereich von 5 bis 10 Gew-%.

Die Herstellung des glasig-kristallinen Materials erfolgt durch Gemengebildung mit geeignete Substanzen, also mit 15 - 45 Gew-% CaO, 40 - 45 Gew-% P₂O₅, 10 - 40 Gew-% ZrO₂ und 0,7 - 3,5 Gew-% Fluorid.Das Fluorid wird vorteilhaft als CaF₂ eingebracht. Die Gemengebestandteile werden miteinander kombiniert und in geeigneten meist mehrstufigen Temperaturbehandlungsprogrammen (Haltestufen im Bereich von 400 bis 1500 °C) in einem geeigneten Tiegelmaterial, vorzugsweise bestehend aus einer Pt/Rh-Legierung, bei 1550 bis 1650 °C zum Schmelzen gebracht. Die Schmelze wird vergossen, und die erstarrte Schmelze wird je nach Verwendungszweck an der Luft (spontane Abkühlung) oder im Kühlofen auf Raumtemperatur abgekühlt. Danach wird der Werkstoff aufgemahlen.

Das so hergestellte glasig-kristalline Material ist ein solches, das einen oder mehrere der folgenden Parameter aufweist
- Gesamtlöslichkeit von 4 bis 5,5 mg/l, wenn die Prüfung in 0,2M TRIS-HCl-Puffer-Lösung bei pH=7,4, T=37 °C, an einer Kornfraktion von 315 - 400 µm, 120h lang bei einem Probenoberflächen-zu-Lösungsmittelvolumen-Verhältnis von 5cm⁻¹ erfolgt,
- thermischer Ausdehnungskoeffizient zwischen 1,4 und 6•10⁻⁶ grad⁻¹ zwischen 27°C und 800°C,
- Stabilität im sauren pH-Bereich zwischen 7,0 und 7,5.

Die hier verwendeten Begriffe "Glaskeramik" und "glasig-kristallines Material", sind im Allgemeinen nicht immer eindeutig definierbar. Es liegen sowohl kristalline als auch glasige bzw. röntgenamorphe Phasen innig vermischt vor. Für die vorliegende Erfindung ist es ohne Belang, ob eine Phase neben der anderen vorliegt oder ob eine Phase die andere umhüllt.

Als "Hauptkristallphase" wird hier eine kristalline Phase bezeichnet, deren Mengenanteil wenigstens doppelt so groß ist, wie die einer Nebenphase, wobei Konzentrationen um 15% und darunter, vorzugsweise unter 10 Gew-% als Nebenphasen bezeichnet werden.

Die Messung der Teilchengröße erfolgt mit der Lasergranulometrie.

Als zusätzlich zu dem genannten glasig-kristallinen Material einsetzbares biokeramisches Material wird vorteilhaft ein Material ausgewählt, das Natrium, Kalium, Calcium, Magnesium, Hydroxyl-Ionen oder Hydroxyl-Bestandteile, Fluorid, Silicat und/oder ortho-Phosphat enthält. Bevorzugt ist ein biokeramisches Material ein solches mit kristallinen Phasen. von Ca₂KNa(PO₄)₂. Der Zusatz von resorbierbaren Biokeramiken bietet die Möglichkeit des Aufbaus poröser Strukturen, die osteokonduktiv und gleichzeitig stützend wirken können. Der Prozeß der Auflösung der Biokeramikpartikelchen ist abhängig von deren Struktur und kann nach Wunsch eingestellt werden. Als vorteilhaft hat sich u.a. ein Material, erwiesen, daß gemäß DE 19744809 C1 hergestellt wurde bzw. Werkstoffe, die Ca₂KNa(PO₄)₂ oder ähnliche Phasen enthalten. Verwendet man hingegen langzeitstabile, bioaktive Keramiken oder Glaskeramiken, so sollte eine der kristallinen Phasen Apatit sein. Als vorteilhaft hat sich eine Glaskeramik auf der Basis Apatit/Wollastonit gemäß DD 247574A3 erwiesen.

Zur Erzielung eines röntgendichteren Werkstoffes empfiehlt es sich, ein Material dem erfindungsgemäß herzustellenden Adhäsiv beizumischen, das aus folgenden Komponenten besteht bzw. sie in Anteilen größer 30 Masse-% enthält, nämlich: CaZr₄(PO₄)₆ und/oder CaTi₄(PO₄)₆. Für den vorliegenden Anwendungsfall ist es von der Sache her gleichgültig, ob Calcium-Zirkonium- und/oder Calcium-Titan-Orthophosphat in amorpher oder der eher typischen kristallinen Form vorliegt.

Es kann weiterhin vorteilhaft sein, daß als zusätzlicher anorganischer Füllstoff TiO₂ vorzugsweise in Mengen von 0,1 bis 10 Gew-% und bevorzugt in der Modifikation Rutil zugegeben werden kann und daß dadurch wesentlich erhöhte Festigkeiten erzielt werden können.

Als biologisch verträgliches zinkhaltiges Pulver wird Zinkoxid oder eine Zinkseife eingesetzt. Die zu den Metallseifen gehörenden Zinkseifen sind Salze mit Resten höherer Fett-, Harz und Naphthensäuren, wie stearate, Palmitate, Oleate, Linoleate, Resinate, Laurate, Octanoate, Ricinoleate, 12-Hydroxystearate, Naphthenate, Tallate usw.

Die Abbindereaktion kann auch über die Bildung von Zn-Seifen sowie den Zutritt von Wasser aus der Umgebung gesteuert werden. Ein Wasserzusatz ist nicht erforderlich.

Auf Grund seiner Struktur weist das Adhäsiv zudem eine Klebrigkeit gegenüber Metalloxiden auf, mit der Folge einer verbesserten Haftung an z.B. keramischen Oberflächen oder Implantaten aus Titanlegierungen mit der jeweils vorgelagerten Oxidschicht.

In das erfindungsgemäße Verfahren miteinbezogen werden können Arzneimittel, wie z.B. Antibiotika, die vorteilhaft einzelnen Mischungskomponenten, z.B. dem glasig-kristallinen oder dem biokeramischen Material zugesetzt werden können oder auch allein in das Gemisch eingebracht werden. Bevorzugt ist Gentamycin mit einem Anteil von etwa 0,5 bis 2 Gew-%, vorzugsweise 0,8 bis 1,3 Gew-%, bezogen auf die Gesamtmasse des Ahäsivs.

Ein besonderer Vorteil des erfindungsgemäßen Adhäsivs besteht darin, daß es sich hierbei um einen monomerfreies Adhäsiv handelt, das einfach anmischbar ist, in seiner Thixotropie und/oder Porengröße einstellbar ist und keine toxischen Substanzen an die Umgebung abgibt. Insbesondere die fehlende Toxizität infolge der Vermeidung von Monomeren sowie üblichen Stabilisatoren und Akzeleratoren ist hervorzuheben. Weiterhin vorteilhaft ist, daß er auch nicht während des Anrührvorgangs zwischen 1 und 10 Minuten, vorzugsweise 4-5 min, aushärtet, und somit eine plastische Phase von durchschnittlich 3 bis 8 min gegeben ist.

Alle diese Faktoren führen zu einer guten Benetzung des Adhäsivs auf einer Metall- oder Keramikoberfläche und somit zu einer gleichmäßigen Schichtdicke. Damit kann ein gleichmäßiger Kontakt zwischen den zu verbindenden Flächen durch das Adhäsiv gewährleistet werden. Verarbeitungsfehler treten dadurch wesentlich vermindert auf.

Der Aushärtevorgang wird durch die Ausbildung von Chelatverbindungen hervorgerufen. Diese können durch Reaktion mit den gegebenenfalls beigemischten Zn²⁺ Ionen, aber auch teilweise mit löslichen Bestandteilen der beiden Keramiken sowie Oberflächenbestandteilen der Fügepartner gebildet werden.

Das erfindungsgemäße temporäre Adhäsiv gestattet die Verbindung von Metall zu Metall, wobei die Metalle gleich oder verschieden sein können, und von Metall zu Keramik für einen begrenzten Zeitraum, wie dies insbesondere in der Stomatologie erwünscht sein kann, beispielsweise beim Verbinden von metallischen Implantaten mit keramischen Kronen oder beim Verbinden von metallischen Implantaten mit Goldkronen. Hierbei ist es oft vorteilhaft, derartige Verbindungen nach mehreren Wochen oder Monaten, teilweise bis zu 2 Jahren, durch den behandelnden Mediziner wieder zu lösen, um bestimmte Wirkungen auf die Implantatumgebung einzuschätzen oder Verunreinigungen im Bereich um und unter der Krone entfernen zu können. Bekannte Knochenzemente oder Haftmittel lassen meist eine gezielte Trennung der Klebeverbindung nicht ohne Beschädigung von Krone, Implantat, umgebendem Zahnfleisch oder mehreren davon zu.

Das erfindungsgemäße Adhäsiv hat Festigkeiten, die den Gebrauch des Zahnersatzes durch den Träger ungehindert gestatten.

Die Erfindung betrifft ferner einen Adhäsiv-Kit auf Basis von Polymethylmethacrylat, gekennzeichnet durch die folgenden, getrennt voneinander vorliegenden Bestandteile
a) 15 bis 50 Gew-% eines monomerfreien Polymethylmethacrylates (PMMA) mit einer mittleren Molmasse von 3000 bis 200000 Dalton und einer Säurezahl von 10 bis 350 mg KOH pro g Polymer ;
b) 5 bis 40 Gew-% eines biologisch verträglichen, organischen Lösungsmittels oder Lösungsmittelgemisches für das PMMA;
c) 0,05 bis 80 Gew-% eines glasig-kristallinen Materials mit einer Korngröße im Bereich von 0,05 bis 20 µm, bestehend aus 15 - 45 Gew-% CaO, 40 - 45 Gew-% P₂O₅, 10 - 40 Gew-% ZrO₂ und 0,7 - 3,5 Gew-% Fluorid und enthaltend als Hauptkristallphasen Apatit und Calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen.

Der Kit kann gegebenenfalls zusätzlich 3 bis 30 Gew-%, bezogen auf das Gesamtgewicht des Kits, eines biologisch verträglichen zinkhaltigen Pulvers und/oder ein resorbierbares biokeramisches Material gemäß DE 197 44 809 oder gemäß EP 0541546 und/oder ein langzeitstabiles biokeramisches Material gemäß DD 247 574 enthalten.

Der Knochenzement-Kit kann weiterhin zusätzlich einzeln oder als Gemisch mit der Komponente c) Anteile an TiO₂ enthalten sowie zusätzlich einzeln oder als Gemisch mit der Komponente c) ein Röntgenkontrastmittel, vorzugsweise bis zu 30 Gew-% CaZr₄(PO₄)₆ oder CaTi₄(PO₄)₆ oder Gemische davon.

Das biologisch verträgliche Lösungsmittel, das zu dem erfindungsgemäßen Adhäsiv-Kit gehört, ist Acetessigsäureethylester oder ein Gemisch von Acetessigsäureethylester mit Ethanol, wobei Ethanol bis zu 4 Vol-% Wasser enthalten kann.

Das biologisch verträgliche zinkhaltige Pulver ist vorteilhaft Zinkoxid oder eine Zinkseife.

Der erfindungsgemäße Kit liegt in sterilisierter Form vor, wobei die Sterilisation durch Ethylenoxid oder mittels Strahlensterilisation vorgenommen werden kann.

Weiterhin kann der Kit Arzneimittelkomponenten im Gemisch mit den Einzelkomponenten oder gesondert enthalten, insbesondere Antibiotika.

Die Erfindung soll nachstehend durch Beispiele näher erläutert werden. Alle Prozentangaben sind auf das Gewicht bezogen.

### Beispiel 1 Herstellung des glasig-kristallinen Materials Apatit/CZP1

Es wird ein Gemenge bereitet, das folgender Zusammensetzung entspricht (Code: Apatit/CZP1):
25,88 CaO
28,44 ZrO₂
43,68 P₂O₅
5,00 CaF₂
Dabei erweist es sich als praktikabel, den CaO-Anteil in Form von 62,79 CaHPO₄ einzusetzen und den noch notwendigen P₂O₅-Anteil in Form von 10,51ml einer 85%igen H₃PO₄. Zunächst wird CaHPO₄, ZrO₂ und CaF₂ gut vermischt, danach mit der Phosphorsäure versetzt, nach Reaktion gemörsert, wobei 4h Trockenhaltestufen von 120°C und 170°C eingelegt wurden. Diese Reaktionsgemisch wird in einem Pt/Rh-Tiegel gefüllt und über die 1h Haltestufen 400 und 800°C erhitzt, abgekühlt und anschließend gemörsert. Das so vorbehandelte Material wird nunmehr im Pt/Rh-Tiegel mit jeweils 15min Haltezeit in den Stufen 800, 1000, 1300, 1500 und schließlich 1600°C zum Schmelzen gebracht und sodann auf eine Stahlplatte (Raumtemperatur) gegossen.

Ein Teil der Schmelze wurde durch Mahlen in einer Achatmühle zerkleinert, unter 43µm abgesiebt und sodann einer röntgendiffraktographischen Untersuchung unterzogen. Das Ergebnis (Röntgendiffraktogramm) zeigt, daß in dem glasig-kristallinen Produkt die Kristallphasen Apatit (Fluorapatit/Hydroxylapatit) und Calciumzirconiumphosphat [CaZr₄(PO₄)₆] deutlich nachweisbar sind. Der restliche Teil der Schmelze wird auf eine Körnung von 0,05-20 µm gebracht.

### Beispiel 2 Herstellung des glasig-kristallinen Materials Apatit/CZP2

Es wird ein Gemenge nach der Vorschrift des Beispiels 1 hergestellt mit dem Unterschied, daß hier als zusätzliche Komponente Natriumoxid eingebracht wird (Code: Apatit/CZP2). Der Ansatz sieht die Verwendung folgender Gemengebestandteile vor:
59,93 CaHPO₄
27,10 ZrO₂
3,42 Na₂O
5,00 CaF₂ und
9,56ml einer 85%igen H₃PO₄-Säure.
Es wurde wie im Beispiel 1 gearbeitet. Nach der letzten Temperaturhaltestufe wurde die Schmelze aus dem Tiegel auf eine Stahlplatte gegossen.

Ein Teil der Schmelze wurde durch Mahlen in einer Achatmühle zerkleinert, unter 43µm abgesiebt und sodann einer röntgendiffraktographischen Untersuchung unterzogen. Das Ergebnis (Röntgendiffraktogramm) zeigt, das in dem glasig-kristallinen Produkt die Kristallphasen Apatit (Fluorapatit/Hydroxylapatit) und Calciumzirconiumphosphat [CaZr₄(PO₄)₆] und Natriumzirconiumphosphat [NaZr₂(PO₄)₃] nachweisbar sind.

Der restliche Teil der Schmelze wird auf eine Körnung von 0,05-20 µm gebracht.

### Beispiel 3 Ausdehnungskoeffizienten Apatit/CZP1

Es erfolgte die Herstellung eines glasig-kristallinen Materials nach Beispiel 1 (Apatit/CZP1). Das Material wird durch Mahlen in einer mit Zirconiumoxid ausgekleideten Mühle zerkleinert, so daß sich ein D₅₀-Wert von 8µm ergab. Das gemahlene Gut wird mit einer 5%igen Polyvinylalkohol(PVA)-Lösung im Verhältnis Mahlgut zu PVA-Lösung von 90 : 10 Masse-% versetzt und zu einem Stab mit 4,7kN in einem Preßgesenk verpreßt. Dieser Rohkörper wird gesintert bei einer Temperatur von 1050°C.

An dem auf diese Weise erhaltenen relativ dichten Formkörper wird der thermische Ausdehnungskoeffizient(AK) bestimmt:
AK im Bereich von 27 - 400°C: 1,90*10⁻⁶ grd Celsius⁻¹
AK im Bereich von 50 - 400°C: 1,86*10⁻⁶ grd Celsius⁻¹
AK im Bereich von 30 - 300°C: 1,45*10⁻⁶ grd celsius⁻¹
AK im Bereich von 30 - 400°C: 1,88*10⁻⁶ grd Celsius⁻¹
AK im Bereich von 30 - 600°C: 2,6*10⁻⁶ grd celsius⁻¹
AK im Bereich von 30 - 800°C: 3,2*10⁻⁶ grd Celsius⁻¹

### Beispiel 4 Chemische Beständigkeit Apatit/CZP1 im basischen Bereich

Es erfolgt die Herstellung eines glasig-kristallinen Materials nach Beispiel 1 (Apatit/CZP1). Sodann wird das Material gemörsert und eine Kornfraktion von 315 - 400µm daraus hergestellt.

Das auf diese Weise erhaltene Granulat wird im Vergleich zu einem Grundglas (Ap40_{Glas}) und einer aus diesem Grundglas hergestellten Glaskeramik auf der Basis von Apatit und Wollastonit (Ap40ₖᵣᵢₛₜ.) [chemische Zusammensetzung also identisch zu (Gewichts-%): 44,3 SiO₂; 11,3 P₂O₅; 31,9 CaO; 4,6 Na₂O; 0,19 K₂O; 2,82 MgO und 4,99 CaF₂] hinsichtlich seiner chemischen Beständigkeit verglichen.

Zu diesem Zweck wurden zunächst die spezifischen Oberflächen nach BET mit Krypton als Meßgas bestimmt zu:
Apatit/CZP1: 0,364m²/g
Ap40_{Glas} : 0,018 m²/g
Ap40_{krist.} : 0,055m²/g.

Hier zeigt sich, daß das im erfindungsgemäßen Adhäsiv eingesetzte glasig-kristalline Material eine gewisse offene Porosität im Vergleich zum Grundglas und der daraus hergestellten Glaskeramik aufweist. Diesen Unterschieden wird bei den Lösungsuntersuchungen dadurch Rechnung getragen, indem das Oberflächen(Proben) zu Lösungsmittelvolumen (TRIS-HCl-Puffer-Lösung) konstant auf 5cm⁻¹ eingestellt wird.

Als Lösungsmittel wurde eine 0,2M TRIS-HCl-Puffer-Lösung mit pH=7,4 bei 37°C verwendet. Die Lagerung erfolgte bei 37°C über eine Zeitdauer von 120h. Danach wurde die Gesamtlöslichkeit durch Bestimmung der Einzelionen (Ca,P, Zr) in der Lösung mit Hilfe einer ICP-Messung bestimmt zu:
Apatit/CZP1: 4,1 - 5,1 mg/L
Ap40_{Glas}: 318 - 320mg/L
Ap40_{krist.}: 75,2 - 82,0 mg/L.

Die Werte belegen eindrucksvoll die hohe chemische Beständigkeit des in dem erfindungsgemäßen Adhäsiv eingesetzten neuen Werkstoffes unter simulierten physiologischen Bedingungen, einer bekannten Methode zur Bestimmung der Langzeitbeständigkeit in vitro.

### Beispiel 5 Chemische Beständigkeit Apatit/CZP1 im sauren Bereich

Es wird vorgegangen wie im Beispiel 4, jedoch wird eine 0,2M TRIS-HCl-Puffer-Lösung mit dem pH-Wert von 6,0 bei 37°C zur Messung verwendet. Auf diese Weise läßt sich der Fall simulieren, daß infolge einer Wundheilungs- oder Spätinfektion der pH-Wert von den physiologischen 7,4 in den sauren Bereich abgleitet.

Mit Hilfe der ICP wurden nun folgende Werte der Gesamtlösung (Ca, P, Zr) bestimmt:
Apatit/CZP1: 16-19 mg/L
Ap40_{Glas}: 505-518 mg/L
Ap40ₖᵣᵢₛₜ : 117-125 mg/L.

Die Werte belegen eindrucksvoll die hohe chemische Beständigkeit des für die Erfindung eingesetzten Werkstoffes unter simulierten Bedingungen, wie sie bei einer Entzündungsreaktion vorliegen. Demzufolge ist die Erhöhung der absoluten Werte der Löslichkeit für den erfindungsgemäßen Werkstoff wesentlich geringer im Vergleich zu der doch dramatischen Erhöhung im Falle des Grundglases bzw. der Glaskeramik auf Apatit/Wollastonit-Basis.

### Beispiel 6 bis 8

Zur Prüfung der Zugfestigkeit wurden auf Ti-Kegelstümpfe mit einer Höhe von 4mm und einem oberen Durchmesser von 3,5mm und einem unteren Durchmesser von 4,5mm Käppchen aus In-Ceram (Vita Zahnfabrik), Galvanogold (Wieland Edelmetalle) sowie aus Empress 2 (Ivoclar) mit einem Adhäsiv, bestehend aus 30% Polymeransatz in einem Gemisch aus 60 Vol-% Ethanol und 40 Vol-% Acetessigsäureethylester unter Zusatz von 35 Gew-% Pulver befestigt. Das Pulver bestand aus ZnO, TiO₂, und einem glasig-kristallinen Material gemäß Beispiel 1. Nach 24h Lagerung in Wasser bei 37°C wurde die Zugfestigkeit durch Auseinanderziehen mit Hilfe einer Universalprüfmaschine Zwick , Vorschub 1 mm/min ermittelt.

| | Beispiel 6 | Beispiel 7 | Beispiel 8 |
|---|---|---|---|
| Kappe | Empress 2 | Galvanogold | In Ceram |
| Zugfestigkeit nach 24 h 37°C Wasserlagerung | 35 N | 131 N | 129 N |
| Nach 10 Wochen Lagerung in Wasser | | 77 N | 78 N |

Gegenüber dem bekannten Harvardzement (Zinkphosphatzement) mit einer Zugfestigkeit von 300-600 N zeigt ein Material wie Empress 2 im Verbund mit Titanium Zugfestigkeiten, die etwa eine Größenordnung niedriger liegen und für eine temporäre Befestigung gut geeignet sind.

## Patentansprüche

1. Verfahren zur Herstellung eines temporären Adhäsivs für Metall-Metall- und Metall-Keramik-Bindungen, **dadurch gekennzeichnet, daß** man
15 bis 50 Gew-%, bezogen auf das Gesamtgewicht des Adhäsivs, eines monomerfreien Polymethylmethacrylates (PMMA) mit einer mittleren Molmasse von 3000 bis 200000 Dalton und einer Säurezahl von 10 bis 350 mg KOH pro g Polymer mit einem biologisch verträglichen, organischen Lösungsmittel oder Lösungsmittelgemisch für das PMMA vermischt, und
der Mischung 0,05 bis 80 Gew-%, bezogen auf das Gesamtgewicht des Adhäsivs, eines bioaktiven, glasig-kristallinen Materials mit einer Korngröße im Bereich von 0,05 bis 20 µm zusetzt, bestehend aus 15 - 45 Gew-% CaO, 40 - 45 Gew-% P₂O₅, 10 - 40 Gew-% ZrO₂ und 0,7 - 3,5 Gew-% Fluorid, bezogen auf das Gesamtgewicht des glasig-kristallinen Materials, und als Hauptkristallphasen Apatit und Calciumzirconiumphosphat und als Nebenbestandteil eine Glasphase aufweisend, worin die Hauptkristallphasen des glasig-kristallinen Materials zusammen wenigstens 35 Gew-% betragen und die Nebenbestandteile 5 bis 15 Gew-% betragen,
unter Rühren und bei einer Temperatur von 10 bis 50 °C bis zum Erhalt einer fließfähigen Mischung mit einer offenen Verarbeitungszeit im Bereich von 1 bis 20 Minuten.

2. Verfahren nach Anspruch 1, **dadurch gekennzeichnet, daß** ein Polymethylmethacrylat mit einem Anteil von 30 bis 35 Gew-% eingesetzt wird.

3. Verfahren nach Anspruch 1, **dadurch gekennzeichnet, daß** als biologisch verträgliches Lösungsmittel Acetessigsäureethylester oder ein Gemisch von Acetessigsäureethylester mit Ethanol, wobei Ethanol bis zu 4 Vol-% Wasser enthalten kann, eingesetzt wird, vorzugsweise Acetessigsäureethylester.

4. Verfahren nach Anspruch 1, **dadurch gekennzeichnet, daß** dem glasig-kristallinen Material eine weitere langzeitstabile oder eine vollständig oder teilweise resorbierbare Biokeramik zugesetzt wird.

5. Verfahren nach Anspruch 4, **dadurch gekennzeichnet, daß** als biokeramisches Material ein resorbierbares mit kristallinen Phasen von Ca₂KNa(PO₄)₂ eingesetzt wird oder eine langzeitstabile Glaskeramik auf der Basis von Apatit/Wollastonit.

6. Verfahren nach Anspruch 1, **dadurch gekennzeichnet, daß** als glasig-kristallines Material ein solches mit der Zusammensetzung 23 - 39 Gew-% CaO, 40 - 45 Gew-% P₂O₅, 20 - 35 Gew-% ZrO₂ und 1 - 3 Gew-% Fluorid eingesetzt wird.

7. Verfahren nach Anspruch 1, **dadurch gekennzeichnet, daß** als glasig-kristallines Material ein solches eingesetzt wird, das zusätzlich 0,1 bis 6 Gew-% Na₂O enthält und zusätzlich als Nebenbestandteil eine Natriumzirconiumphosphat-Phase enthält.

8. Verfahren nach Anspruch 1, **dadurch gekennzeichnet, daß** den Ausgangskomponenten oder Gemischen davon ein Arzneimittel zugesetzt wird.

9. Verfahren nach Anspruch 1, **dadurch gekennzeichnet, daß** als weiterer anorganischer Füllstoff TiO₂ zugegeben wird, vorzugsweise 0,1 bis 10 Gew-% und weiterhin bevorzugt in der Modifikation Rutil.

10. Verfahren nach Anspruch 1, **dadurch gekennzeichnet, daß** dem Gemisch zusätzlich 3 bis 30 Gew-% eines biologisch verträglichen zinkhaltigen Pulvers zugesetzt wird, insbesondere Zinkoxid oder eine Zinkseife.

11. Verfahren nach Anspruch 1, **dadurch gekennzeichnet, daß** zur Erzielung eines röntgendichten Adhäsivs bis zu 30 Gew-% CaZr₄ (PO₄)₆ oder CaTi₄ (PO₄)₆ oder Gemische davon oder Mischkristalle daraus zugegeben werden.

12. Verfahren nach einem der Ansprüche 1 bis 11, **dadurch gekennzeichnet, daß** die Viskosität des Adhäsivs über den Anteil der Mischungskomponenten und/oder das Molekulargewicht des PMMA eingestellt wird.

13. Adhäsiv-Kit auf Basis von Polymethylmethacrylat, **gekennzeichnet durch** die folgenden, getrennt voneinander vorliegenden Bestandteile
a) 15 bis 50 Gew-%, bezogen auf das Gesamtgewicht des Adhäsivs, eines monomerfreien Polymethylmethacrylates (PMMA) mit einer mittleren Molmasse von 3000 bis 200000 Dalton und einer Säurezahl von 10 bis 350 mg KOH pro g Polymer ;
b) 10 bis 40 Gew-%, bezogen auf das Gesamtgewicht des Adhäsivs, eines biologisch verträglichen, organischen Lösungsmittels oder Lösungsmittelgemisches für das PMMA;
c) 0,05 bis 80 Gew-%, bezogen auf das Gesamtgewicht des Adhäsivs, eines bioaktiven, glasig-kristallinen Materials mit einer Korngröße im Bereich von 0,05 bis 20 µm, bestehend aus 15 - 45 Gew-% CaO, 40 - 45 Gew-% P₂O₅ , 10 - 40 Gew-% ZrO₂ und 0,7 - 3,5 Gew-% CaF₂, bezogen auf das Gesamtgewicht des glasig-kristallinen Materials, und als Hauptkristallphasen Apatit und Calciumzirconiumphosphat und als Nebenbestandteil eine Glasphase aufweisend, worin die Hauptkristallphasen des glasig-kristallinen Materials zusammen wenigstens 35 Gew-% betragen und die Nebenbestandteile 5 bis 15 Gew-% betragen.

14. Adhäsiv-Kit nach Anspruch 14, **dadurch gekennzeichnet, daß** er zusätzlich einen Bestandteil enthält, ausgewählt aus der Gruppe, bestehend aus TiO₂, Röntgenkontrastmittel wie CaZr₄ (PO₄)₆ oder CaTi₄(PO₄)₆, ein resorbierbares biokeramisches Material mit kristallinen Phasen von Ca₂KNa(PO₄)₂, eine langzeitstabile Glaskeramik auf der Basis Apatit/Wollastonit oder Gemische davon.

15. Adhäsiv-Kit nach Anspruch 13, **dadurch gekennzeichnet, daß** er zusätzlich 3 bis 30 Gew-% eines biologisch verträglichen zinkhaltigen Pulvers enthält, vorzugsweise Zinkoxid oder eine Zinkseife.

16. Adhäsiv-Kit nach Anspruch 13, **dadurch gekennzeichnet, daß** das biologisch verträgliche Lösungsmittel Acetessigsäureethylester oder ein Gemisch von Acetessigsäureethylester mit Ethanol ist, wobei Ethanol bis zu 4 Vol-% Wasser enthalten kann, vorzugsweise Acetessigsäureethylester.

## Claims

1. A method for the production of a temporary adhesive for metal-metal and metal-ceramic bonds comprising the steps of mixing 15 to 50% by weight, relative to the total weight of the adhesive, of a monomer-free polymethylmethacrylate (PMMA) whose average molar mass ranges between 3,000 and 200,000 daltons and whose acid value ranges between 10 and 350mg KOH per g polymer with a biocompatible, organic solvent or solvent mixture for the PMMA, and
adding 0.05 to 80% by weight, relative to the total weight of the adhesive, of a bioactive vitreous-crystalline material with a particle size ranging between 0.05 and 20µm to the mixture, which material consists of 15-45% by weight CaO, 40-45% by weight P₂O₅, 10-40% by weight ZrO₂ and 0.7-3.5% by weight fluoride, relative to the total weight of the vitreous-crystalline material, and comprises apatite and calcium zirconium phosphate as main crystal phases and a glass phase as an auxiliary component, said main crystal phases of the vitreous-crystalline material jointly making up at least 35% by weight and said auxiliary components making up 5 to 15% by weight,
while stirring and at a temperature ranging between 10 and 50°C until a flowable mixture is obtained whose open processing time ranges between 1 an 20 minutes.

2. A method according to Claim 1, wherein a polymethylmethacrylate is used in an amount ranging between 30 and 35% by weight.

3. A method according to Claim 1, wherein the biocompatible solvent used is ethyl acetoacetate or a mixture of ethyl acetoacetate with ethanol, which ethanol may contain water up to an amount of 4% by volume, preferably ethyl acetoacetate.

4. A method according to Claim 1, wherein another long-term stable or a totally or partially resorbable bioceramic is added to the vitreous-crystalline material

5. A method according to Claim 4, wherein the bioceramic material used is a resorbable material comprising crystalline phases of Ca₂KNa(PO₄)₂ or a long-term stable glass ceramic based on apatite/wollastonite.

6. A method according to Claim 1, wherein the vitreous-crystalline material used is a material composed of 23-39% by weight CaO, 40-45% by weight P₂O₅, 20-35% by weight ZrO₂ and 1-3% by weight fluoride.

7. A method according to Claim 1, wherein the vitreous-crystalline material used is a material additionally containing 0.1 to 6% by weight Na₂O and additionally containing a sodium zirconium phosphate phase as an auxiliary component.

8. A method according to Claim 1, wherein a medicine is added to the starting components or mixtures thereof.

9. A method according to Claim 1, wherein TiO₂ is added as an additional inorganic filler, preferably in an amount ranging between 0.1 and 10% by weight and preferably in the form of its modification rutile.

10. A method according to Claim 1, wherein in addition 3 to 30% by weight of a biocompatible powder containing zinc is added to the mixture, particularly zinc oxide or a zinc soap.

11. A method according to Claim 1, wherein CaZr₄ (PO₄) ₆ or CaTi₄(PO₄)₆ or mixtures thereof or mixed crystals therefrom is/are added in an amount ranging up to 30% by weight in order to obtain an X-ray dense adhesive.

12. A method according to any of Claims 1 through 11, wherein the viscosity of the adhesive is adjusted via the amount of the components making up the mixture and/or the molecular weight of the PMMA.

13. An adhesive kit based on polymethylmethacrylate comprising the following components provided separate of one another:
a) 15 to 50% by weight, relative to the total weight of the adhesive, of a monomer-free polymethylmethacrylate (PMMA) whose average molar mass ranges between 3,000 and 200,000 daltons and whose acid value ranges between 10 and 350mg KOH per g polymer;
b) 10 to 40% by weight, relative to the total weight of the adhesive, of a biocompatible organic solvent or solvent mixture for the PMMA;
c) 0.05 to 80% by weight, relative to the total weight of the adhesive, of a bioactive, vitreous-crystalline material with a particle size ranging between 0.05 and 20µm consisting of 15-45% by weight CaO, 40-45% by weight P₂O₅, 10-40% by weight ZrO₂ and 0.7-3.5% by weight CaF₂, relative to the total weight of the vitreous-crystalline material, and comprising apatite and calcium zirconium phosphate as main crystal phases and a glass phase as an auxiliary component, said main crystal phases of the vitreous-crystalline material jointly making up at least 35% by weight and said auxiliary components making up 5 to 15% by weight.

14. An adhesive kit according to Claim 13, wherein said kit additionally contains a component selected from the group consisting of TiO₂, an X-ray contrast medium such as CaZr₄(PO₄)₂ or CaTi₄ (PO₄) ₆, a resorbable bioceramic material comprising crystalline phases of Ca₂KNa(PO₄)₂, a long-term stable glass ceramic based on apatite/wollastonite or mixtures thereof.

15. An adhesive kit according to Claim 13, wherein said kit additionally contains 3 to 30% by weight of a biocompatible powder containing zinc, preferably zinc oxide or a zinc soap.

16. An adhesive kit according to Claim 13, wherein the biocompatible solvent used is ethyl acetoacetate or a mixture of ethyl acetoacetate with ethanol, which ethanol may contain water up to an amount of 4% by volume, preferably ethyl acetoacetate.

## Revendications

1. Procédé pour la production d'un adhésif temporaire pour des liaisons métal-métal et métal-céramique, **caractérisé en ce que** :
on mélange 15 à 50 % en poids, par rapport au poids total de l'adhésif, d'un polyméthylméthacrylate (PMMA) exempt de monomère dont la masse moléculaire moyenne se situe entre 3 000 et 200 000 daltons et dont l'indice d'acidité se situe entre 10 et 350 mg de KOH par g de polymère avec un solvant ou un mélange de solvants pour le PMMA, organique et biocompatible, et
on ajoute au mélange 0,05 à 80 % en poids, par rapport au poids total de l'adhésif, d'un matériau bioactif, vitro-cristallin présentant une taille de particule située entre 0,05 et 20 µm, constitué de 15 à 45 % en poids de CaO, de 40 à 45 % en poids de P₂O₅, de 10 à 40 % en poids de ZrO₂ et de 0,7 à 3,5 % en poids de fluorure, par rapport au poids total du matériau vitro-cristallin, et comprend de l'apatite et du phosphate de zirconium aluminium en tant que phases cristallines principales et une phase vitreuse en tant que composant auxiliaire, lesdites phases cristallines principales du matériau vitro-cristallin représentant conjointement au moins 35 % en poids et lesdits composants auxiliaires représentant 5 à 15 % en poids,
tout en agitant et à une température de 10 à 50 °C jusqu'à l'obtention d'un mélange fluide dont le temps ouvert se situe entre 1 et 20 minutes.

2. Procédé selon la revendication 1, **caractérisé en ce qu'**un polyméthylméthacrylate est utilisé en une quantité située entre 30 et 35 % en poids.

3. Procédé selon la revendication 1, **caractérisé en ce que**, comme solvant biocompatible, on utilise de l'acétoacétate d'éthyle ou un mélange d'acétoacétate d'éthyle et d'éthanol, lequel éthanol peut contenir de l'eau jusqu'à 4 % en volume, de préférence de l'acétoacétate d'éthyle.

4. Procédé selon la revendication 1, **caractérisé en ce qu'**on ajoute au matériau vitro-cristallin une autre biocéramique stable à long terme ou totalement ou partiellement résorbable.

5. Procédé selon la revendication 4, **caractérisé en ce que**, comme matériau biocéramique, on utilise un matériau résorbable avec des phases cristallines de Ca₂KNa(PO₄)₂ ou une vitrocéramique à stabilité à long terme basée sur l'apatite/wollastonite.

6. Procédé selon la revendication 1, **caractérisé en ce que**, comme matériau vitro-cristallin, on utilise un tel matériau avec une composition de 23 à 39 % en poids de CaO, de 40 à 45 % en poids de P₂O₅, de 20 à 35 % en poids de ZrO₂ et de 1 à 3 % en poids de fluorure.

7. Procédé selon la revendication 1, **caractérisé en ce que**, comme matériau vitro-cristallin, on utilise un tel matériau, qui contient en outre 0,1 à 6 % en poids de Na₂O et qui contient en outre comme composant auxiliaire une phase de phosphate de zirconium-sodium.

8. Procédé selon la revendication 1, **caractérisé en ce qu'**un produit pharmaceutique est ajouté aux composants de départ ou aux mélanges de ceux-ci.

9. Procédé selon la revendication 1, **caractérisé en ce que**, comme charge inorganique additionnelle on ajoute du TiO₂, de préférence en quantité de 0,1 à 10 % en poids et de préférence sous la forme modifiée de rutile.

10. Procédé selon la revendication 1, **caractérisé en ce que** 3 à 30 % en poids d'une poudre biocompatible contenant du zinc, en particulier de l'oxyde de zinc ou un savon de zinc est ajouté en plus au mélange.

11. Procédé selon la revendication 1, **caractérisé en ce qu'**une quantité jusqu'à 30 % en poids de CaZr₄(PO₄)₆ ou CaTi₄(PO₄)₆ ou des mélanges de ceux-ci ou des cristaux mixtes de ceux-ci est ajoutée de façon à obtenir un adhésif opaque aux rayons X.

12. Procédé selon l'une quelconque des revendications de 1 à 11, **caractérisé en ce que** la viscosité de l'adhésif est ajustée par la quantité des composants constituant le mélange et/ou par la masse moléculaire du PMMA.

13. Kit d'adhésif basé sur du polyméthylméthacrylate **caractérisé par** les composants suivants, présentés indépendamment les uns des autres :
a) 15 à 50 % en poids, par rapport au poids total de l'adhésif, d'un polyméthylméthacrylate (PMMA) exempt de monomère dont la masse moléculaire moyenne se situe entre 3 000 et 200 000 daltons et dont l'indice d'acidité se situe entre 10 et 350 mg de KOH par g de polymère
b) 10 à 40 % en poids, par rapport au poids total de l'adhésif, d'un solvant ou un mélange solvant organique biocompatible pour le PMMA ;
c) 0,05 à 80 % en poids, par rapport au poids total de l'adhésif, d'un matériau bioactif, vitro-cristallin de taille de particule située entre 0,05 et 20 µm, lequel matériau est composé de 15 à 45 % en poids de CaO, de 40 à 45 % en poids de P₂O₅, de 10 à 40 % en poids de ZrO₂ et de 0,7 à 3,5 % en poids de fluorure, par rapport au poids total du matériau vitro-cristallin, et comprend de l'apatite et du phosphate de zirconium aluminium en tant que phases cristallines principales et une phase vitreuse en tant que composant auxiliaire, lesdites phases cristallines principales du matériau vitro-cristallin représentant conjointement jusqu'au moins 35 % en poids et lesdits composants auxiliaires représentant jusqu'à 5 à 15 % en poids.

14. Kit d'adhésif selon la revendication 13, **caractérisé en ce qu'**il contient un composant, choisi dans le groupe composé de TiO₂, un produit de contraste de rayon X tel que le CaZr₄(PO₄)₆ ou CaTi₄ (PO₄) ₆, un matériau biocéramique résorbable avec des phases cristallines de Ca₂KNa(PO₄)₂,une vitrocéramique stable à long terme basée sur l'apatite/wollastonite ou des mélanges de ceux-ci.

15. Kit adhésif selon la revendication 13, **caractérisé en ce qu'**il contient de 3 à 30 % en poids d'une poudre biocompatible contenant du zinc, de préférence de l'oxyde de zinc ou un savon de zinc.

16. Kit adhésif selon la revendication 13, **caractérisé en ce que** le solvant biocompatible est de l'acétoacétate d'éthyle ou un mélange d'acétoacétate d'éthyle et d'éthanol, lequel éthanol peut contenir de l'eau jusqu'à une quantité de 4 % en volume, de préférence de l'acétoacétate d'éthyle.
